# EUROPEAN PATENT APPLICATION

(11) **EP 4 312 228 A1**
(43) Date of publication of application: **31.01.2024**
(21) Application number: 22186686.6
(22) Date of filing: 25.07.2022
(51) Int. Cl.: G16H 50/20, A61B 5/00, A61B 5/024, A61B 5/11, A61B 5/1455

(54) **SYSTEM FOR SUPPORTING A PATIENT'S HEALTH CONTROL AND OPERATING METHOD OF SUCH SYSTEM**

(71) Applicant: BIOTRONIK SE & Co. KG, 12359 Berlin (DE)
(72) Inventor: Ciesla, Catharina-Sophie, 12167 Berlin (DE); Hackebeil, Alexander, 12623 Berlin (DE)
(74) Representative: Biotronik Corporate Services SE

(57) **Abstract**

The invention comprises a system for supporting a patient's health control as well as a method of operating such system, wherein the system comprises a wearable device (A) and at least one remote device (B, S, R), wherein the wearable device (A) comprises a first communication module (220, 230) and each one of the at least one remote device (B, S, R) comprises a second communication module, wherein the wearable device (A) is configured to collect pre-defined physiological data of the patient from at least two sender devices (E, M, P) and to analyze the collected physiological data based on a pre-defined first analysis scheme and to generate an actual health status information of the patient based on this analysis, wherein the wearable device (A) is further configured to automatically transmit the actual health status information of the patient to the respective second communication module of one or several pre-defined remote devices (B, S, R) of the at least one remote device (B, S, R) using the first communication module (220, 230). The invention further comprises respective computer program product and computer readable data carrier.

## Description

The invention is directed to a system for supporting a patient's health control comprising a wearable device and at least one remote device and to a respective operating method of such system method as well as a to computer program product and to a computer readable data carrier. The computer program product may be a software routine, e.g. related to hardware support means within the at least one remote device and/or the wearable device.

Wearable devices or medical devices such as cardio messengers, emergency watches, receivers for wireless insulin pumps and blood glucose meters are known. Each one of these devices produces or analyses data of the patient, in particular physiological data. Often, these data are derivable from the respective device. When received from the respective device the patient or a health care practitioner (HCP) may assess these data but as these data are separate from each other it is difficult for the HCP to get a condensed overview of such data, to recognize an emergency information and to start appropriate measures relating to such emergency information.

It is therefore desirable to have a system that provides a more sophisticated monitoring of the patient's health status, either on a regular basis or on demand by the patient or by a pre-defined person such as an HCP or a relative.

The above problem is solved by a system for supporting a patient's health control comprising a wearable device and at least one remote device with the features of claim 1, by a method for operating such system with the features of claim 7, by a computer program product with the features of claim 13, and by a computer readable data carrier with the features of claim 14.

In particular, the above problem is solved by a system for supporting a patient's health control comprising a wearable device and at least one remote device, wherein the wearable device comprises a first communication module and each one of the at least one remote device comprises a second communication module, wherein the wearable device is configured to collect pre-defined physiological data of the patient from at least two sender devices and to analyze the collected physiological data based on a pre-defined first analysis scheme and to generate an actual health status information of the patient based on this analysis, wherein the wearable device is further configured to automatically transmit the actual health status information of the patient to the respective second communication module of one or several pre-defined remote devices of the at least one remote device using the first communication module.

Sender devices may be any kind of sensors including also wearable, non-medical sensing devices such as fitness tracker, body scale, etc. Furthermore, a sender device may be any device (e.g. smartphone) with an app (fitness apps. health apps, etc.).

The above system creates an interface which is formed by the wearable device that communicates with sender devices which may be common medical devices, commercial health supporting devices and/or active implants. The wearable device is worn close to the patient's body and makes the physiological data of the patient available to remote devices such as specific mobile devices or devices at authorized locations. However, according to the invention, not the vast amount of physiological data is directly transmitted to the at least one remote device but a (short and/or easily understandable) information on the actual, i.e., most recent, health status of the patient such as "Everything is in order." or "Attention. High blood pressure. Please check the medicine and contact your doctor, if necessary." or "Attention. Diabetic patient. Dangerously low blood sugar value detected. Please contact the patient and emergency service.". In one embodiment, some selected physiological data which might be interesting for the treating HCP and/or trusted person and/or relative may be transmitted along with the actual health status information of the patient.

The patient wears the wearable device and the wearable device is used to monitor the health status of the patient. The wearable device comprises a first communication module having a transceiver for receiving physiological data from the at least two sender devices and for transmitting the actual health status information of the patient to the at least one remote device. Further, the wearable device may comprise a processor for analyzing collected physiological data and/or a data memory to store the received physiological data. Accordingly, the first communication module, the processor and the data memory may be connected for data exchange. The wearable device may comprise a battery, for example a rechargeable battery.

The physiological data collected by the wearable device from the at least two sender devices may include, but are not limited to, ECG, impedance, activity, posture, heart sounds, blood pressure, oxygen saturation, blood sugar and/or respiration data. Physiological data may be any type of bodily parameter measurement value that may be derived by any type of sensor from the patient's body. Additionally, physiological data are also data which are not directly measurable but may be calculated from the measured data, for example a mean value of a pre-defined parameter value with regard to a pre-defined time period, e.g., the mean value per day, or the variability of any physiological data throughout a pre-defined time period, e.g., the heart rate variability. Such derived data are also referred to as physiological data in the following.

Each remote device is a functional unit that is located within some, if applicable a considerable, distance from the wearable device and that can perform (e.g., by a processor) substantial computations, including numerous arithmetic operations and logic operations without human intervention, such as, for example, a personal mobile device (PMD), a smartphone, a handheld device, a desktop computer, a server computer, clusters/warehouse scale computer and/or embedded system. The remote device may be or may comprise, for example, a mobile device (e.g., a smartphone) and/or a stationary remote device operated by a clinic or the HCP. Each remote device may be a single device or a computer system having several units. In case, the remote device is a computer system, the remote device may comprise a relay or intermediate device and a central server, wherein the relay device transmits the actual health status information of the patient to the central computer facility. The mobile device of the remote device may comprise an interface which may be used by the HCP, another person of trust and/or a relative of the patient to provide input data. If the remote device comprises several separate units, they are configured to communicate with each other using respective further communication modules. The functionality of the remote device may be realized as an application implemented in the remote device (e.g., a smartphone). The remote device may belong to a pre-defined contact person of the patient and/or a emergency station and/or a clinic and, accordingly, may be operated by the pre-defined contact person and/or an HCP working at the emergency station or the clinic.

Each of the at least one remote device and the wearable device comprises at least one processor which executes instructions comprising an instruction control unit and an arithmetic and logic unit.

The communication connection between the remote device and the wearable device is provided by the first communication module and the second communication module. The first communication module of the wearable device may establish a communication channel to the second communication module of the remote device at pre-defined time intervals or on demand (triggered). A communication module of the at least two sender devices provides (one-directional) data transmission of physiological data of the patient to the remote device and/or the wearable device. The second communication module of the remote device may be bi-directional for transmission of the actual health status information of the patient and a trigger signal to be sent to the wearable device. In one embodiment data exchange of the wearable device and the at least two sender devices may be bi-directional, as well. Such communication comprises in most cases communication over the air (i.e. wireless, without wire) and/or may be provided by wired media. The communication may use inductive magnetic means, acoustic methods (e.g. ultrasound), and/or acoustic, optical and/or electromagnetic waves, for example Bluetooth, WLAN, ZigBee, NFC, Wibree or WiMAX in the radio frequency region, Ethernet, or IrDA or free-space optical communication (FSO) in the infrared or optical frequency region. Communication may be provided using the internet, telecommunication networks and/or wireless networks, wherein the actual health status information and/or telephone calls and/or speech and/or text messages may be transmitted.

Each of the remote device, at least two sender devices and the wearable device may further comprise a data memory which may include any volatile, non-volatile, magnetic, or electrical media, such as a random access memory (RAM), read-only memory (ROM), non-volatile RAM (NVRAM), electrically-erasable programmable ROM (EEPROM), flash memory, or any other memory device. In the data memory the collected physiological data and/or the first and/or second analysis scheme and/or health status information of the patient may be stored permanently and/or temporarily.

In one embodiment of the system, the wearable device is configured such that the actual health status information of the patient is transmitted to the respective second communication module of the pre-defined remote device
- at pre-defined time point and/or
- after receipt of a respective trigger signal provided by the patient or provided by one remote device and/or
- in case the analysis of the collected data revealed that the physiological data of the patient meet a pre-defined emergency condition.
Accordingly, the actual health status information may be generated and transmitted at a pre-defined time point, e.g., in regular pre-defined time intervals and/or upon request, i.e. after receipt of a respective trigger signal, wherein the trigger signal is provided by the patient or a pre-defined contact person or the HCP using the respective remote device and/or if a pre-defined emergency condition is met.

In the latter case, in one embodiment, the processor of the wearable device may determine whether a pre-defined physiological data type crosses a pre-defined emergency threshold value or not, wherein the emergency threshold value may be defined by the HCP or the patient reflecting the pre-defined emergency condition. Using a threshold value as a trigger (when it is crossed) for transmitting a health status information to the second communication module of one (or several) pre-defined remote device(s) is an easy and fast determinable way of informing the pre-defined user of an assumed emergency situation of the patient.

There may be several possible scenarios for the analysis: In a first scenario a threshold analysis is performed. Here, the threshold value may be dynamically adjusted to the mean value of a defined observation period or time interval. In a second scenario both a single exceedance and a defined number of exceedances in a defined time interval may be used for triggering when a threshold is exceeded. In a similar third scenario, the occurrence of a sudden large deviation from the mean or the last n measured values may be a trigger. The same applies to the absence/presence of measurement data of predefined parameters (blood glucose, blood pressure, etc.).

In one embodiment of the system, the wearable device is configured such that the trigger signal from the remote device and/or the physiological data from the at least two sender devices are wirelessly received and/or the actual health status information is wirelessly transmitted.

In one embodiment of the system, the at least two sender devices are devices of the group comprising at least one medical sensor and at least one medical device. In the above system the medical device may be, for example, a pacemaker, a cardiac monitor, an ILP, a defibrillator, an ILPS, an ICD, a blood glucose sensor, a long term ECG-recorder, an O2 sensor, an optical pulse sensor, an insulin pump and/or a neurostimulator. The medical device may be an implanted medical device which is fully or at least partly implanted within the patient's body. The medical device may be also a (medical) device worn on the surface/skin of the body. The at least two sender devices may transmit physiological data to the wearable device using its respective communication module in pre-defined regular time intervals and/or upon request by the wearable device (e.g., if the patient has triggered the wearable device and thereby activated determination and transmission of his/her actual health status information).

In one embodiment of the system, the at least two sender devices additionally comprise at least one device of the group comprising least one further sensor and at least one further device, for example a positioning sensor or a temperature sensor, wherein the further sensor or the further device provide further, non-physiological data to the wearable device, wherein the wearable device is configured to analyze the further data together with the collected physiological data based on a pre-defined second analysis scheme and to generate an actual health status information of the patient based on such analysis of the collected physiological data and the collected further data. Non-exhaustive examples of further data are bed time, rise time, activity level throughout the day or within a pre-defined period of the day, posture information during usual sleep time period, activity during usual sleep time period, activity level, walking gait, posture, temperature of the environment, geographical position. One or several sender devices forming the further sensor or the further device may be a component separate from or integrated within the wearable device. The further sensor or further device may be realized in form of an app implemented at the wearable device or another (mobile) device.

Additionally or alternatively, the wearable device itself may contain a positioning sensor or a temperature sensor so that a further external sensor is not mandatory.

In one embodiment of the system, the trigger signal may comprise an information defining the receiving remote device and/or an information defining a type of first analysis scheme or a type of second analysis scheme on which the determined actual health status information is based. This means that the trigger signal may define the receiving person and/or institution which requests the actual health status information of the patient. For example, the trigger signal may contain the information, that the actual health status information may be transmitted to the respective remote device of a pre-defined relative and/or a pre-defined clinic. Further, if the trigger signal additionally or alternatively comprises the type of first analysis scheme or the type of second analysis scheme, the content of the actual health status information to be transmitted may be defined. For example, the clinic may be interested in a more detailed actual health status information covering the heart frequency whereas a more general actual health status information (e.g. "the patient is well") may be transmitted to the remote device of the pre-defined relative. Accordingly, a first type of first/second analysis scheme may analyze the collected physiological data different from a second type of first/second analysis scheme (e.g. by a different algorithm).

According to the invention, the collected physiological data of the patient and, if applicable, further non-physiological data are processed by the processor of the wearable device using a pre-defined analysis scheme and the actual health status information of the patient is determined. The analysis scheme may comprise two analyzing steps. In a first step received values may be analyzed individually according to the described metrics, e.g. exceeding or deviation of a measured value from the defined range. In a second step, an analysis of defined combinations of measured values (e.g. heart rate and exercise, blood glucose level and weight, etc.) may be performed with regard to certain criteria, e.g. trend progression. The data processing produces actual and general health status information such as "Everything is in order." or "Attention. High blood pressure. Please check the medicine and contact your doctor, if necessary." or "Attention. Diabetic patient. Dangerously low blood sugar value detected. Please contact the patient and emergency service.". Such information is very helpful for the pre-defined contact person or the HCP to have a quick overview on the actual situation of the patient. The contact person or HCP may request further patient's data from the wearable device, if necessary.

The above problem is further solved by method of operating a system for supporting a patient's health control comprising a wearable device and at least one remote device, wherein the wearable device comprises a first communication module and each one of the at least one remote device comprises a second communication module, comprising the following steps:
- Collecting pre-defined physiological data of the patient from at least two sender devices by the wearable device,
- Analyzing the collected physiological data based on a pre-defined first analysis scheme and generating an actual health status information of the patient based on this analysis by the wearable device,
- Automatically transmitting the actual health status information of the patient to the respective second communication module of one or several pre-defined remote devices of the at least one remote device using the first communication module of the wearable device.

In one embodiment of the method, the actual health status information of the patient is transmitted to the respective second communication module of the pre-defined remote device
- at pre-defined time point and/or
- after receipt of a respective trigger signal provided by the patient or provided by one remote device and/or
- in case the analysis of the collected data revealed that the physiological data of the patient meet a pre-defined emergency condition.

In one embodiment of the method, the trigger signal and/or the physiological data from the at least two sender devices are wirelessly received and/or the actual health status information is wirelessly transmitted.

In one embodiment of the method, the trigger signal from the remote device and/or the physiological data from the at least two sender devices are wirelessly received and/or the actual health status information is wirelessly transmitted.

In one embodiment of the method, the at least two sender devices are devices of the group comprising at least one medical sensor and at least one medical device.

In one embodiment of the method, the at least two sender devices additionally comprise at least one device of the group comprising least one further sensor and at least one further device, for example a positioning sensor or a temperature sensor, wherein the further sensor or the further device provide further data to the wearable device, wherein the wearable device analyzes the further data together with the collected physiological data based on a pre-defined extended analysis scheme and generates an actual health status information of the patient based on this analysis of the collected physiological data and the collected further data.

In one embodiment of the method, the trigger signal comprises an information defining the receiving remote device and/or an information defining a type of first analysis scheme or a type of second analysis scheme.

The method steps of the above-mentioned methods are explained above in connection with the system. It is referred to above system explanation with regard to the method steps.

The above method is, for example, realized as a computer program (to be executed at or within the wearable device and/or the remote device, in particular utilizing their processors) which is a combination of above and below specified (computer) instructions and data definitions that enable computer hardware or a communication system to perform computational or control functions and/or operations, or which is a syntactic unit that conforms to the rules of a particular programming language and that is composed of declarations and statements or instructions needed for an above and below specified function, task, or problem solution.

Furthermore, a computer program product is disclosed comprising instructions which, when executed by a processor, cause the processor to perform the steps of the above defined method. Accordingly, a computer readable data carrier storing such computer program product is described.

The present invention will now be described in further detail with reference to the accompanying schematic drawing, wherein
- Fig. 1: shows a first embodiment of a system for supporting a patient's health control,
- Fig. 2: depicts one embodiment of the wearable device with a charging device in a side view, and
- Fig. 3: shows the units of the electronic components of the wearable device of Fig. 2.

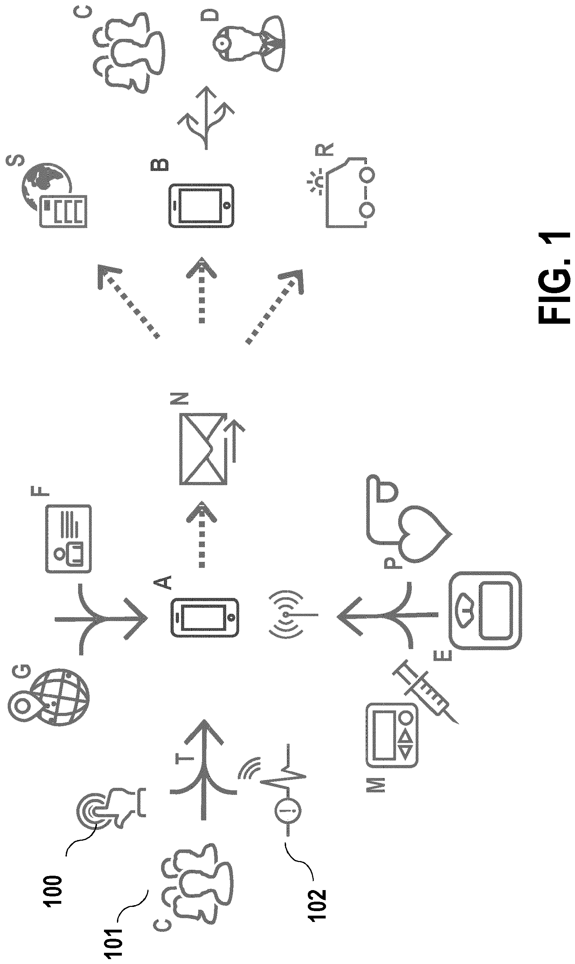

The system of Fig. 1 comprises a wearable device A which is wearable on the patient's body and a remote device B, S, R, for example using a wristband, a belt clip or a chain. The wearable device A has a first communication module and the remote device B, S, R comprises a second communication module, wherein the first communication module and the second communication module are configured to receive and transmit data and messages via common wireless communication methods, e.g. Bluetooth and telecommunication networks. The wearable device A acts as an interface by collecting physiological data from multiple senders, e.g. from medical sensors M fixed to the patient's body (e.g. an insulin pump), implants P (e.g. a pacemaker) implanted within the patient's body as well as from external medical and/or nonmedical devices E of the patient, e.g. a blood glucose meter and/or an activity monitor. The physiological data of the senders M, P, E are then analyzed by a pre-defined first analysis scheme and an actual health status information is generated, representing the actual health condition of the patient. Any new health status information overwrites the previous health status information stored in a data memory of the wearable device A. When triggered by a trigger T, the actual health status information is transmitted in a message N to the remote device B in form of a handheld device, to the remote device S in form of a service center and/or to a remote device R (e.g., a computer) accommodated by an emergency rescue. The service center S may include a server, cloud storage or other online services for data storage. The data may include not only physiological data received from the sender E, M, P but also non-physiological general data F about the patient and the patient's actual health status information.

Once the physiological data has been retrieved, it may be either sent directly to the receiver device or analyzed, depending on the configuration. The data analysis is performed in two steps, first individual measured values are checked against defined metrics (threshold, deviation, etc...). In a second step, combinations of certain measured values are considered together, e.g. trend analysis, so that a deterioration in the state of health may be detected at an early stage, before critical values are exceeded. The data analysis is performed regularly and on trigger.

In one embodiment, some environmental data G, such as position of the patient and temperature in the surrounding of the patient may be received by the first communication unit of the wearable device A as further, non-physiological data, and may be processed by the analysis scheme and thereby included in the determination of the patient's actual health status information.

Triggering T may be provided manually (see reference number 100) by the patient using, for example, a trigger knob or switch area at the surface/display of the wearable device A, and/or by an external request (see reference number 101) and/or automatically during analysis of the physiological data received from the pre-defined sender devices (see reference number 102 in Fig. 1), if the processor of the wearable device A reveals during data analysis a pre-defined emergency condition, for example low blood glucose level in combination with a high heart rate or high blood pressure in combination with low heart rate. In addition to triggered transmission of the data, regular transmission of physiological data and/or current health status information may also be provided at a defined time interval using the first communication module of the wearable device A.

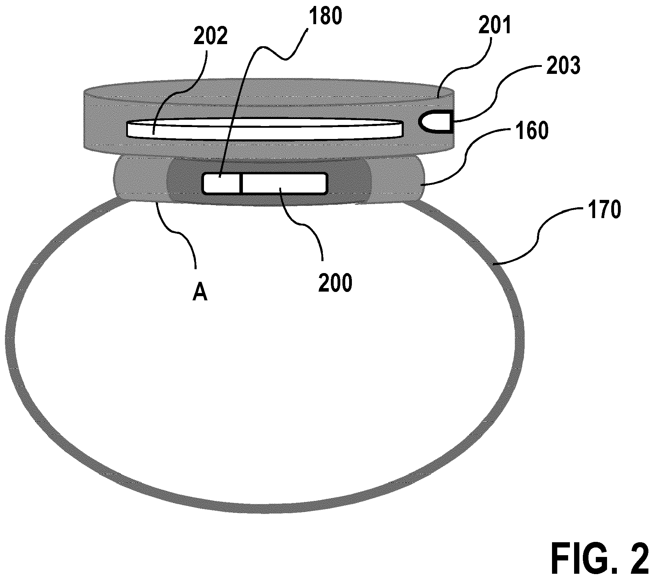

An embodiment of the wearable device A is shown in Fig. 2. The wearable device comprises a body 160 and a wristband 170, wherein the wristband 170 may be used to fix the wearable device A at the patient's arm. Within the body 160 the wearable device A accommodates a rechargeable battery 200 that may be charged via a charging device 201. Further, the body 160 of the wearable device A may comprise further electronic components 180 that are described below. The electronic components are located within the body 160. The charging device 201 may be located on top of the wearable device A as depicted in Fig. 2. Charging is provided contactless - inductively or using charging/conducting contacts. The charging device 201 comprises a battery 202 that can be charged via a USB. This allows the wearable device A to be charged while being worn or while connecting the charging device 201 to an external power supply using socket 203.

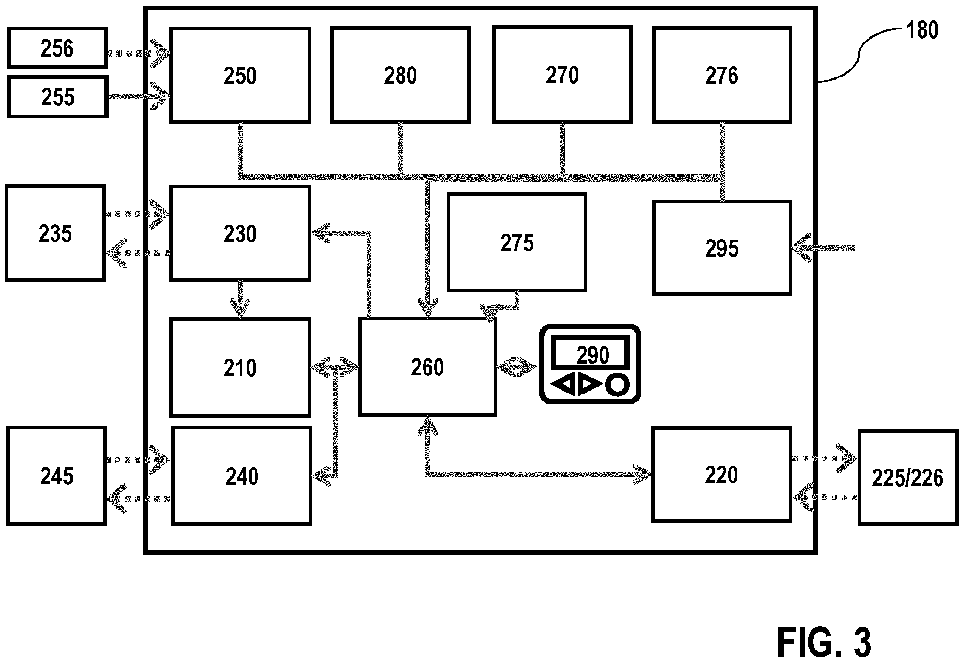

The electronic components 180 of the wearable device A are shown schematically in Fig. 3 and comprise a data memory 210 where received physiological data received from the at least one sender devices E, M, P may be stored permanently as well as temporarily. Additionally, the data memory 210 may store information regarding the data analysis as well as a pre-defined emergency condition and/or at least one pre-defined analysis scheme. The electronic components 180 further comprise the first communication module that is capable of receiving and/or transmitting of messages and/or data, in particular physiological data and, if applicable, and/or further information and the health status information of the respective patient, for the mobile network, mobile internet as well as wireless networks. The first communication module may comprise a telephone communication unit 220 that is configured to send and receive calls 225 as well as (voice) messages 226. Further, the first communication module may comprise a data communication unit 230 for bidirectional communication of data 235 with the remote devices B, S, R as well as senders of physiological data (e.g. external medical devices E, implants P, medical sensors M). In one embodiment, the electronic components 180 may comprise an audio unit 240, which makes it possible to record and/or play back speech or tones 245, for example, of the patient. The electronic components 180 may further comprise a triggering unit 250 that may be actuated manually 255 on the wearable device A by the patient. Further, a software/communication based triggering 256 of the triggering unit 250 that is described below may alternatively or additionally be used.

The electronic components 180 may further include a computing unit/processor 260 that provides data processing and controlling of all data and information received and transmitted as described above and below. Further, a clock 275 may be provided for time determination, wherein in one embodiment it may comprise an alarm and/or reminder function that can be read/notified visually or by voice. In one embodiment, a position sensor 270 (e.g. using GPS or comparable methods) may be provided as a part of the electronic components 180 of the wearable device A. In one embodiment the electronic components 180 may comprise an inclination and/or acceleration sensor 276. In addition or alternatively, they may comprise one or more further sensors 280 for collecting medical data, such as pulse, oxygen saturation, blood pressure as well as environmental data in the form of temperature, magnetic field, humidity, etc.

In one embodiment the electronic components 180 may comprise a display or touch display 290. Accordingly, the wearable device A may be operated using voice commands via the audio unit 240, using touch control via the touch display 290 and/or via physical control. The electronic components 180 may comprise an optical, thermal and/or biometric sensor 295 that can be used to provide information for control functions of the wearable device A. The body 160 may be configured such that it is waterproof, so it can be worn while showering/bathing as well as in humid environments without impairing the functions of the wearable device A.

The units 210, 220, 230, 240, 250, 270, 275, 276, 280, 290, 295 of the electronic components 180 are all connected to the processor 260 as indicated by the arrows in Fig. 3.

The remote device B, S, R may be implemented in various ways, as a standalone mobile or permanent device or as a system. It may comprise an application for realization of the software functions. In particular, the remote device B, S, R is configured to receive the actual health status of the patient generated by the processor 260 of the wearable device A. In one embodiment the remote device B, S, R may comprise a unit for addressing the wearable device A, which may be implemented in hardware and/or via direct contact of the wearable device A and the remote device B, S, R or contactless (e.g. in the form of wireless communication by sending a signal or message). Each of the remote device B, S, R comprises a second communication module that allows to receive data/messages and signals from the wearable device A, in particular the data representing the actual health status of the patient.

The unit for addressing the wearable device A may be a trigger unit which reacts in different ways, i.e. depending on which trigger signal is sent, and/or on the scope and pre-processing of the data varies. The advantage is that the current measured values of existing sensors may be viewed both remotely and at the patient (e.g. when the nursing or rescue service visits), or the rescue service may obtain information about the exact condition of the patient as soon as the alarm is raised.

"Direct contact of the wearable device A and the remote device B, S, R" means that it may be queried not only remotely, but also by placing a receiver device on the wearable (like programming head & pacemaker principle). Furthermore, it offers the possibility to enable or change certain functions only in the physical presence of the remote device (e.g. change of trigger thresholds only by a physician).

Additionally, it may comprise a transmitter to transmit the received data and information to another remote device and/or to transmit a trigger signal to the wearable device A requesting the actual health status of the patient. The remote device B, S, R may further comprise a data memory for temporary and/or permanent storage of the received data/messages and signals. Alternatively or additionally, it may have a USB interface through which the data memory may be read out.

To receive the actual health status information, in one embodiment, the wearable device A needs to be triggered. The triggering T for querying this data is carried out via manual triggering or via a virtual trigger. Manual triggering is performed by the patient by operating, e.g. a knob or a switch area at the wearable device A. Virtual triggering is provided by receiving an external, wirelessly transmitted request, for example in the form of a message from the external device B, S, R. The message may contain a security code by which the wearable device A recognizes the triggering. The request may be actively sent by an authorized contact person C or the responsible HCP D via the respective remote device B, S, R or directly via a message containing the corresponding security code. Via the contact details of the authorized contact person stored in the data memory 210 of the wearable device A or via a security code previously shared by the patient with his HCP D or contact person C, the request authenticates itself as authorized. Different security codes may be used for authorized contact persons C and the HCP D, so that different actual health status information 340 may be generated. Similarly, virtual triggering may be provided in the event that the received physiological data reveal that the health status of the patient meets a pre-defined emergency condition.

After a trigger T has been triggered, the wearable device A queries all or a pre-defined number of the pre-defined sender devices E, M, P. To do this, it sends pre-defined queries to, for example, the respective implants P, external medical devices E, medical sensors M to receive physiological data and, for example, to the patient's mobile device in order to query some further data G from an app, e.g. position data or temperature data. These data are analyzed by the processor 260 of the wearable device A using a pre-defined analysis scheme which is explained above. Thereby an actual health status information is created and packaged into a message N which is then sent to the remote device B and/or S and/or R using wireless communication.

The actual health status information may also contain the patient data F stored in the wearable device A or further, non-physiological data G with regard to the patient and a short but exact characterization of the actual health status of the patient such as "The patient XXX has low blood sugar of YYY and needs immediate help!", or "The patient XXX is well.". The data format of the message N containing the actual health status information conform with the communication method that is used to transmit the message N from the wearable device A to the remote device B, R, S.

The configuration of the wearable device A may be provided using an application, which may be executed on a mobile device or on a computer. The application may include the following functions:
- Recording of patient data F and information to be stored on the wearable device A which may include information regarding diseases, allergies, diagnoses, medication intake, treating physicians, emergency contacts, organ donation, living will, etc.
- Definition of the authorized contact persons C as well as their respective remote devices, wherein the personal information of the contact persons may comprise the name, a telephone number and/or an email address.
- Implementation of the security codes for data retrieval from the senders/other devices M, E, P, G, and communication with the respective remote devices B, R, S.
- Definition of the sender devices to be read out, such as implants P, medical sensors M and external medical devices E, with which the wearable device A is to communicate and of the physiological data to be received from these senders.
- Definition of applications on the patient's mobile devices (application of measuring devices, fitness trackers, health apps, etc.) from which further data G is to be requested.
- Definition of regular queries of the linked apps as well as of the sender devices in pre-defined time intervals and respective updates of the physiological and other data. For this purpose, the wearable device A sends respective requests to the sender devices and/or other devices and starts the synchronization of the data. The request is, for example, provided using wireless communication.
- The definition of the emergency condition(s) that lead(s) to automatic triggering of the wearable device A, e.g., a limit value of physiological data.

In one embodiment, at regular intervals, the wearable device A sends a request to the sender devices E, M, P to transmit the most actual physiological data of the patient. Additionally, the wearable device A may also query the most actual further, non-physiologic data from the application running at the wearable device A at pre-defined time intervals. These data may be processed in the processor 260 using the above explained analysis scheme that considers the physiological data and further data of the app (e.g. position data, temperature data).

The authorized contact persons C and the wearable device A may exchange calls, messages or voice messages via the mobile network or wireless networks at any time. The HCP D and the authorized contact persons C may send a virtual trigger signal using their remote device B, R, S at any time directly to the wearable device A using their second communication module to query the health status of the patient. The latter is notified by the wearable device A about the request and may add an additional comment to the message N containing the data.

If the processor 260 of the wearable device A reveals any pre-defined emergency condition during processing of the received physiological and other data a virtual trigger is activated, as well. For example, a visual and/or audible indication may be provided on the wearable device and at the same time all available data is combined to a message N. Immediately, the message N is created containing the actual health status information of the patient and sent to the pre-defined remote devices B, R, S as indicated above.

In some embodiments, the wearable device may be also a portable device (e.g. smartphone, tablet, etc.).

In the event of sudden discomfort or an incident the patient may request transmission of the actual health status information and thereby may request help using the wearable device A by manually activating the trigger T at the wearable device A.

There may be different profiles for recipient remote devices in the case of different events. For example, if a contact person requests the health status of the patient by sending a virtual trigger, the actual health status information may be sent to the respective remote device B of the requesting contact person, only. However, in the event of a mechanical trigger by the patient or a virtual trigger based on a pre-defined emergency condition, the actual health status information may be sent to a greater number of remote devices, e.g. to the remote device B of the authorized contact person C, to a remote device in form of a service center S and/or directly to a remote device at an emergency center R. Accordingly, the emergency rescue team may already obtain an overview of the patient's condition on their way to the patient and prepare suitable therapy measures.

In an embodiment, if emergency rescue is alerted conventionally and upon arrival the rescue team finds that the patient is wearing a wearable device A having above functionality, the emergency rescue team may directly query the actual health status information of the patient using a respective remote device R.

The advantage of the system and method is that all available data on the patient's condition and important information in the form of diagnosed illnesses, medications and allergies is sent immediately when an emergency is triggered. This saves valuable time, so that the person rushing to help or emergency rescue can get an idea of the patient's condition in advance. In the case of a diabetic, for example, this information is passed on directly so that the rescue service or any assisting person knows that the affected person suffers from diabetes and that this may be the cause of the current emergency call. Another advantage is for the pre-defined contact person having the pre-defined remote device (e.g. a relative or other responsible person of the patient). The person receives health status information of the patient at any time so that it is ensured at all times that the patient receives help immediately, if it is necessary. In particular, this system offers chronically ill patients more certainty in everyday life. For example, parents of chronically ill children, for example in the case of diabetes, may supervise remotely the blood glucose level as well as insulin doses and help if necessary.

## Claims

1. A system for supporting a patient's health control comprising a wearable device (A) and at least one remote device (B, S, R), wherein the wearable device (A) comprises a first communication module (220, 230) and each one of the at least one remote device (B, S, R) comprises a second communication module, wherein the wearable device (A) is configured to collect pre-defined physiological data of the patient from at least two sender devices (E, M, P) and to analyze the collected physiological data based on a pre-defined first analysis scheme and to generate an actual health status information of the patient based on this analysis, wherein the wearable device (A) is further configured to automatically transmit the actual health status information of the patient to the respective second communication module of one or several pre-defined remote devices (B, S, R) of the at least one remote device (B, S, R) using the first communication module (220, 230) .

2. The system of claim 1, wherein the wearable device (A) is configured such that the actual health status information of the patient is transmitted to the respective second communication module of the pre-defined remote device (B, S, R)
• at pre-defined time point and/or
• after receipt of a respective trigger signal (T) provided by the patient or provided by one remote device (B, S, R) and/or
• in case the analysis of the collected data revealed that the physiological data of the patient meet a pre-defined emergency condition.

3. The system of any one of the previous claims, wherein the wearable device (A) is configured such that the trigger signal (T) from the remote device (B, S, R) and/or the physiological data from the at least two sender devices (E, M, P) are wirelessly received and/or the actual health status information is wirelessly transmitted.

4. The system of any one of the previous claims, wherein the at least two sender devices (E, M, P) are devices of the group comprising at least one medical sensor and at least one medical device.

5. The system of any one of the previous claims, wherein the at least two sender devices (E, M, P) additionally comprise at least one device of the group comprising least one further sensor and at least one further device, for example a positioning sensor or a temperature sensor, wherein the further sensor or the further device provide further data to the wearable device (A), wherein the wearable device (A) is configured to analyze the further data (G) together with the collected physiological data based on a pre-defined second analysis scheme and to generate an actual health status information of the patient based on this analysis of the collected physiological data and the collected further data (G).

6. The system of any one of the previous claims, wherein the trigger signal comprises an information defining the receiving remote device (B, S, R) and/or an information defining a type of first analysis scheme or a type of second analysis scheme.

7. A method of operating a system for supporting a patient's health control comprising a wearable device (A) and at least one remote device (B, S, R), wherein the wearable device (A) comprises a first communication module (220, 230) and each one of the at least one remote device (B, S, R) comprises a second communication module, comprising the following steps:
- Collecting pre-defined physiological data of the patient from at least two sender devices (E, M, P) by the wearable device (A),
- Analyzing the collected physiological data based on a pre-defined first analysis scheme and generating an actual health status information of the patient based on this analysis by the wearable device (A),
- Automatically transmitting the actual health status information of the patient to the respective second communication module of one or several pre-defined remote devices (B, S, R) of the at least one remote device (B, S, R) using the first communication module (220, 230) of the wearable device (A).

8. The method of claim 7, wherein the actual health status information of the patient is transmitted to the respective second communication module of the pre-defined remote device (B, S, R)
• at pre-defined time point and/or
• after receipt of a respective trigger signal (T) provided by the patient or provided by one remote device (B, S, R) and/or
• in case the analysis of the collected data revealed that the physiological data of the patient meet a pre-defined emergency condition.

9. The method of any one of the claims 7 to 8, wherein the trigger signal (T) from the remote device (B, S, R) and/or the physiological data from the at least two sender devices (E, M, P) are wirelessly received and/or the actual health status information is wirelessly transmitted.

10. The method of any one of the claims 7 to 9, wherein the at least two sender devices (E, M, P) are devices of the group comprising at least one medical sensor and at least one medical device.

11. The method of any one of the claims 7 to 10, wherein the at least two sender devices (E, M, P) additionally comprise at least one device of the group comprising least one further sensor and at least one further device, for example a positioning sensor or a temperature sensor, wherein the further sensor or the further device provide further data (G) to the wearable device (A), wherein the wearable device (A) analyzes the further data (G) together with the collected physiological data based on a pre-defined second analysis scheme and generates an actual health status information of the patient based on this analysis of the collected physiological data and the collected further data.

12. The method of any one of the claims 7 to 11, wherein the trigger signal comprises an information defining the receiving remote device (B, S, R) and/or an information defining a type of first analysis scheme or a type of second analysis scheme.

13. A computer program product comprising instructions which, when executed by a processor, causes the processor to perform the steps of the method according to any one of the claims 7 to 12.

14. Computer readable data carrier storing a computer program product according to claim 13.
